# EUROPÄISCHE PATENTANMELDUNG

(11) **EP 3 789 044 A1**
(43) Veröffentlichungstag der Anmeldung: **10.03.2021**
(21) Anmeldenummer: 20202728.0
(22) Anmeldetag: 28.10.2016
(51) Int. Cl.: A61K 49/10

(54) **VERFAHREN ZUR HERSTELLUNG VON PHARMAZEUTISCHEN FORMULIERUNGEN ENTHALTEND CHELATKOMPLEXE VON GADOLINIUM MIT REDUZIERTEM GEHALT AN TOXISCHEN VERUNREINIGUNGEN**

(62) Teilanmeldung aus: 16196240.2
(71) Anmelder: B.E. Imaging GmbH, 76534 Baden-Baden (DE)
(72) Erfinder: Feuerstein, Jürgen, D-82205 Gilching (DE)
(74) Vertreter: Drescher, Christian

(57) **Zusammenfassung**

Die vorliegende Erfindung betrifft ein Verfahren zur Herstellung von flüssigen zur parenteralen Verabreichung geeigneten pharmazeutischen Formulierungen enthaltend Gadolinium-Chelatkomplexe, insbesondere von Gadolinium-DOTA, mit reduziertem Gehalt an toxischen Verunreinigungen zur Verwendung als Kontrastmittel für die Magnetresonanzbildgebung.

## Beschreibung

Verfahren zur Herstellung von pharmazeutischen Formulierungen enthaltend Chelatkomplexe von Gadolinium mit reduziertem Gehalt an toxischen Verunreinigungen.

### HINTERGRUND DER ERFINDUNG

Chelatkomplexe von Lanthaniden als paramagnetischen Metallen, insbesondere Gadolinium, werden in Form von flüssigen parenteralen pharmazeutischen Formulierungen seit Jahren als Kontrastmittel z.B. für die Magnetresonanztomographie verwendet.

Zu diesem Zweck sind zahlreiche Präparate auf Basis von Gadolinium-Komplexen zugelassen, beispielweise Magnevist® (Gd-DTPA), Primovist® (Gd-EOB-DTPA), Gadovist® (Gd-BT-DO3A), ProHance® (Gd-HP-DO3A), Omniscan® (Gd-DTPA-BMA), OptiMARK® (Gd-DTPA-BMEA), MultiHance® (BOPTA) oder Dotarem® (Gd-DOTA).

Die Tagesdosen von gadoliniumhaltigen Komplexen sind mitunter beträchtlich - so beträgt beispielsweise die maximale Tagesdosis von Gadolinium bei der Gabe von Gadolinium-DOTA 11,7 g, ggf. sogar darüber.

Gadolinium-haltige Chelat-Komplexe wie Gd-DOTA werden in der Regel durch Zusammenfügen von Gadoliniumoxid (Gd₂O₃) und Chelat-Komplexbildner in Wasser hergestellt. Für die o.g. Tagesdosis von Gadolinium-DOTA werden umgerechnet 3,8g Gd₂O₃ eingewogen.

Durch die bergmännische Gewinnung bedingt können mit dem Gadoliniumoxid gadoliniumähnliche Verunreinigungen, insbesondere Schwermetalle, in die dem Patienten verabreichten Gadolinium-Chelat-Komplexe in beträchtlicher Menge eingeschleppt werden, was mehrere Probleme nach sich zieht.

Erstens sind gadoliniumähnliche Verunreinigungen wie Cadmium (Cd), Quecksilber (Hg), Kobalt (Co), Blei (Pb) oder Arsen (As) teilweise extrem toxisch, Arsen und Cadmium zudem genotoxisch.

Zweitens bilden weitere gadoliniumähnliche, an sich weniger giftige, Verunreinigungen wie beispielsweise Zink (Zn) mit Chelatbildnern wie beispielsweise DOTA, DTPA und dessen Derivate, D03A und dessen Derivate oder BOPTA äußerst stabile Komplexe mit hoher Komplexbildungs-Konstante und sind daher in der Lage, Gadolinium aus seinen Komplexen zu verdrängen.

Freie, also nicht komplexierte, Ionen von Gadolinium sind aber äußerst giftig.

Erst in 2007 wurde beispielsweise eine Krankheit mit dem Namen nephrogene systemische fibrose (NSF) bekannt, eine schwerwiegende Erkrankung mit folgenreichen Komplikationen bei der Gabe von Kontrastmitteln in der Magnetresonanz-Tomographie insbesondere bei Patienten mit Niereninsuffizienz.

Es wird angenommen, dass NSF zumindest teilweise auf Ablagerungen von freiem Gadolinium im Organismus zurückzuführen sein könnte. In letzter Zeit wurde auch von Gadolinium-Ablagerungen in Hirn und Leber berichtet, deren Auswirkungen bisher allerdings unbekannt sind.

Die derzeit vermarkteten Gadolinium-Chelat-Komplexe sind zwar thermodynamisch sehr stabil, neigen jedoch unter physiologischen Bedingungen dazu, Gadolinium freizusetzen. Dabei scheinen insbesondere sogenannte Transmetallierungs-Reaktionen eine Rolle zu spielen. Diese Transmetallierungs-Reaktionen verlaufen allgemein nach folgender Formel:

GdL + M²⁺ ⇄ Gd³⁺ + [ML]⁻

wobei Gd für Gadolinium, M für Metall; L für Ligand steht.

Da die Gadolinium-Chelat-Komplexe keine kovalenten Bindungen aufweisen, stehen sie im Prinzip mit den freien Ionen im Gleichgewicht. Das hat zur Folge, dass sich dieses Gleichgewicht in Richtung freier Gadolinium-Ionen verschiebt, je höher die Konzentration von freien Metall-Kationen [M⁺] im Blut ist.

Während das bei Elementen wie beispielsweise Eisen weitgehend unproblematisch ist, da der menschliche Körper eine Vielzahl von Mechanismen wie Eisen-Bindungsproteine besitzt, um freies Eisen-Kationen aktiv zu binden, ist dies beim Element Zink kaum bis gar nicht der Fall.

Viele Menschen nehmen, bedingt durch Nahrungsergänzungsmittel, schon recht hohe Mengen an Zink zu sich. Daher sollte es vermieden werden, die Menge an Zink durch Verunreinigungen in Präparaten wie Kontrastmitteln für die Magnetresonanzbildgebung unnötig noch weiter zu erhöhen.

Zwar ist Zink an sich weniger toxisch als beispielsweise Gadolinium (die sichere tägliche Höchstmenge für Zink liegt gemäß Festlegung der European Food Safety Authority (EFSA) bei 25 mg täglich), es bildet aber mit Komplexbildnern, wie sie bei Kontrastmitteln für die Magnetresonanzbildgebung zum Einsatz kommen, ähnlich stabile Chelatkomplexe wie Gadolinium. Dadurch ist Zink in der Lage, das viel toxischere Gadolinium aus seinen Komplexen zu verdrängen.

Um diese unerwünschte Freisetzung von Gadolinium zu verhindern, wird üblicherweise Chelat-Komplexbildner im Überschuss zugegeben. Dieser Überschuss muss einerseits groß genug sein, damit sich die gewünschte Wirkung, d.h. die weitestgehende Komplexierung von Gadolinium-Ionen, einstellt. Er darf andererseits aber auch nicht zu groß sein, da die üblichen Chelat-Komplexbildner oft selber giftig sind.

Die vorliegenden Erfindung stellt nun ein Verfahren zur Herstellung von flüssigen zur parenteralen Verabreichung geeigneten pharmazeutischen Formulierungen enthaltend Gadolinium-Chelatkomplexe mit reduziertem Gehalt an toxischen Verunreinigungen sowie solchen Verunreinigungen, die Gadolinium leicht aus seinen Komplexen verdrängen können, zur Verfügung.

### BESCHREIBUNG DER ERFINDUNG

Im Rahmen der vorliegenden Erfindung bedeutet der Begriff "freier Chelat-Komplexbildner" jenen Komplexbildner, der nicht mit Gadolinium komplexiert ist.

Im Rahmen der vorliegenden Erfindung bedeutet der Begriff "freies Gadolinium" jenes Gadolinium, welches nicht mit einem Chelat-Komplexbildner komplexiert ist.

Desweiteren beziehen sich im Rahmen der vorliegenden Erfindung alle Mengenangaben in ppm auf den Gehalt pro Masse.

Im Rahmen der vorliegenden Erfindung bezeichnet der Begriff "reduzierter Gehalt an toxischen Verunreinigungen" im Zusammenhang mit erfindungsgemäßen flüssigen parenteralen pharmazeutischen Formulierungen enthaltend Gadolinium-Chelatkomplexe solche Formulierungen, deren Gehalt an Zink kleiner als 50 ppm, deren Gehalt an Arsen kleiner als 3 ppm, deren Gehalt an Blei, Kobalt sowie Cadmium jeweils kleiner als 1,5 ppm und der Gehalt an Quecksilber kleiner als 1,0 ppm ist.

Die vorliegende Erfindung betrifft ein Verfahren zur Herstellung von flüssigen parenteralen pharmazeutischen Formulierungen enthaltend Gadolinium-Chelatkomplexe mit reduziertem Gehalt an toxischen Verunreinigungen, umfassend
i. das Vermischen einer vorbestimmten Menge von Gadoliniumoxid mit einer vorbestimmten Menge Chelat-Komplexbildner in Wasser,
ii. das Einstellen des pH-Wertes des in Schritt i. erhaltenen Gemisches auf einen ersten pH-Wert im Bereich von pH 7,7 bis pH 9,0 durch Zugabe einer Base, und anschließende
iii. Absenkung des pH-Wertes des in Schritt iii. erhaltenen Gemisches auf einen zweiten pH-Wert im Bereich von pH 6,5 bis pH 7,5 durch Zugabe einer Säure.
   Das Verfahren ist überdies dadurch gekennzeichnet, dass der Chelat-Komplexbildner in Schritt i. mit der in Schritt iii. verwendeten Säure identisch ist.

Es wurde dabei gefunden, dass eine Erhöhung des pH-Werte über pH 9,0 dazu führt, dass für die anschließende Absenkung des pH-Wertes überproportional viel Säure zugegeben werden muss, was zu unerwünscht hohen Anteilen an freier Säure führt.

In einer bevorzugten Ausführungsform der Erfindung wird das in Schritt i. erhaltene Gemisch im Schritt ii. durch Zugabe einer Base auf einen ersten pH-Wert in einem Bereich von pH 7,9 bis pH 8,3 eingestellt und das in Schritt ii. erhaltene Gemisch im Schritt iii. durch Zugabe einer Säure auf einen pH-Wert in einem Bereich von pH 6,8 bis pH 7,4 eingestellt.

Bevorzugte in Schritt i. verwendete Chelat-Komplexbildner sowie in Schritt iii. verwendete Säuren sind die offenkettigen Komplexliganden DTPA und dessen Derivate und BOPTA sowie die makrocyclischen Komplexliganden D03A und dessen Derivate sowie DOTA. Besonders bevorzugter Chelat-Komplexbildner bzw. Säure ist DOTA.

Besonders bevorzugte flüssige, parenterale, pharmazeutische Formulierungen enthalten das Megluminsalz des Gadolinium-DOTA-Komplexes.

| **Chelat-Komplexbildner** | **Strukturformel** |
|---|---|
| DTPA | 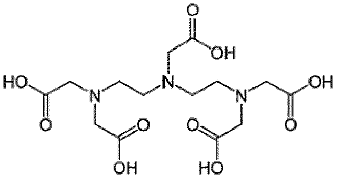 |
| BOPTA | 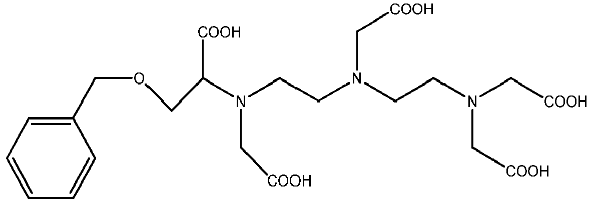 |
| D03A | 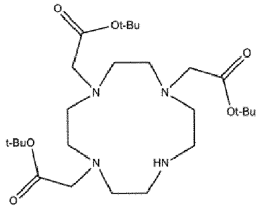 |
| DOTA | 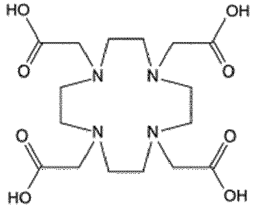 |

Bevorzugte Base für die Durchführung von Schritt ii. ist Meglumin (N-Methylglucamin).

Eine besonders bevorzugte Ausführungsform des Verfahrens umfasst die folgenden Schritte:
i. das Vermischen einer vorbestimmten Menge von Gadoliniumoxid mit einer vorbestimmten Menge DOTA in Wasser,
ii. das Einstellen des pH-Wertes des in Schritt i. erhaltenen Gemisches auf einen ersten pH-Wert im Bereich von pH 7,9 bis pH 8,3 durch Zugabe von Meglumin, und
iii. die Absenkung des pH-Wertes des in Schritt ii. erhaltenen Gemisches auf einen zweiten pH-Wert im Bereich von pH 6,8 bis pH 7,4 durch Zugabe von DOTA.

Mit dem erfindungsgemäßen Herstellungsverfahren werden flüssige zur parenteralen Verabreichung geeignete Formulierungen enthaltend Chelatkomplexe von Gadolinium mit einem Arsengehalt von weniger als 3,0 ppm, einem Gehalt an Blei, Kobalt sowie Cadmium von jeweils weniger als 1,5 ppm und einem Gehalt an Quecksilber von weniger als 1,0 ppm zur Verfügung gestellt.

Darüber hinaus können mit dem erfindungsgemäßen Verfahren flüssige zur parenteralen Verabreichung geeignete Formulierungen enthaltend Chelatkomplexe von Gadolinium mit einem Gehalt an Zink von weniger als 50 ppm zur Verfügung gestellt werden.

Desweiteren wird durch die vorliegende Erfindung eine flüssige zur parenteralen Verabreichung geeignete Formulierung enthaltend einen Komplex eines Chelat-Komplexbildners mit Gadolinium sowie freien Chelat-Komplexbildner bereitgestellt, die weniger als 3,0 ppm Arsen, jeweils weniger als 1,5 ppm Cadmium, Kobalt und Blei, weniger als 1,0 ppm Quecksilber und weniger als 50 ppm Zink enthält.

In bevorzugten Ausführungsformen der Erfindung enthält die flüssige zur parenteralen Verabreichung geeignete pharmazeutische Formulierung jeweils weniger als 1,0 ppm, ganz besonders bevorzugt weniger als 0,5 ppm an Arsen, Blei, Kobalt und Cadmium sowie weniger als 10 ppm, insbesondere weniger als 5 ppm an Zink.

Bevorzugter Chelat-Komplexbildner ist der makrocyclische Chelat-Komplexbildner DOTA. Damit ist der besonders bevorzugte Chelat-Komplex Gadolinium-DOTA.

Besonders bevorzugte flüssige zur parenteralen Verabreichung geeigneten pharmazeutische Formulierungen enthalten das Megluminsalz des Gadolinium-DOTA-Komplexes.

Die erfindungsgemäßen flüssige zur parenteralen Verabreichung geeigneten pharmazeutische Formulierungen finden als Kontrastmittel für die Magnetresonanzbildgebung Verwendung.

### BEISPIELE

### Beispiel 1 - Risikobetrachtung

Die Berechnung der maximalen täglichen Exposition jeder Verunreinigung erfolgte auf der Grundlage der Akzeptanzgrenze für jede Verunreinigung unter der Annahme einer maximalen täglichen Verabreichung von 11,731 g Gadolinium-DOTA.

*** bezogen auf 70 kg Körpergewicht Tabelle 1**

| Inhaltsstoff | Menge per mL | Maximale Tagesdosis* |
|---|---|---|
| Gd-DOTA | 0,27932 g | 11,731 g |
| davon eingewogen als DOTA | 0,20246 g | 8,503 g |
| davon eingewogen als Gadoliniumoxid (Gd₂O₃) | 0,09062 g | 3,806 g |

Die maximal mögliche parenterale Aufnahme der elementaren Verunreinigungen wurde, jeweils bezogen auf die maximale Tagesdosis von Gd₂O₃ unter Berücksichtigung der jeweils verwandten Verbindung sowie deren jeweiligen oberen Akzeptanzgrenze berechnet. Durch Vergleich mit der zulässigen täglichen Exposition gemäß ICH Q3D erfolgte eine Einschätzung des Risikos für jede einzelne elementare Verunreinigung.

| Verbindung | Akzeptanzgrenze | Berechnete maximale parenterale Aufnahme 1µg/Tag]¹⁾ | Element | Klasse ¹⁾ | Berechnete maximale parenterale Aufnahme Element 1µg/Tag]¹⁾ | maximale zulässige parenterale AufnahMe²⁾ [µg/Tag] | Risiko? |
|---|---|---|---|---|---|---|---|
| | | | | | | | |
| As | < 20 ppm | 76,0 | As | 1 | 76,0 | 15 | Ja |
| Cd | < 2 ppm | 7,6 | Cd | 1 | 7,6 | 6 | Ja |
| Hg | < 2 ppm | 7,6 | Hg | 1 | 7,6 | 4 | Ja |
| PbO₂ | < 4 ppm | 15,2 | Pb | 1 | 13,2 | 5 | Ja |
| CoO | < 5 ppm | 19,0 | Co | 2A | 14,9 | 5 | Ja |

| Zum Vergleich: | | | | | | | |
|---|---|---|---|---|---|---|---|
| Se | < 2 ppm | 7,6 | Se | 2A | 7,6 | 85 | Nein |

| | | | | | | | |
|---|---|---|---|---|---|---|---|
| ¹⁾ bezogen auf 3,806 g Gd₂O₃; ²⁾ gemäß ICH Q3D (Version 12/2014) | | | | | | | |

### Beispiel 2 - Herstellungsprozess für Gadotersäure-Meglumin gemäß der vorliegenden Erfindung:

i. Wasser zur Injektion wird in einem Herstellungsbehälter auf 80°C erhitzt und eine vorbestimmte Menge DOTA zugegeben,
ii. Eine vorbestimmte Menge Gadoliniumoxid (Gd₂O₃) wird zur in i. erhaltenen Lösung hinzugegeben und gerührt, bis es vollständig in Lösung geht,
iii. Die in ii. erhaltene Lösung wird auf Raumtemperatur abgekühlt und mittels Meglumin auf einen pH-Wert zwischen pH 7,7 und pH 9,0 eingestellt,
iv. Der pH-Wert der in iii. erhaltenen Lösung wird durch Zugabe von DOTA auf einen Wert von pH 6,5 bis pH 7,5 eingestellt,

## Patentansprüche

1. Verfahren zur Herstellung von flüssigen zur parenteralen Verabreichung geeigneten pharmazeutischen Formulierungen enthaltend Gadolinium-Chelatkomplexe mit reduziertem Gehalt an toxischen Verunreinigungen, umfassend die folgenden Schritte:
i. das Vermischen einer vorbestimmten Menge von Gadoliniumoxid mit einer vorbestimmten Menge von Chelat-Komplexbildner in Wasser,
ii. das Einstellen des pH-Wertes des in Schritt i. erhaltenen Gemisches auf einen ersten pH-Wert im Bereich von pH 7,7 bis pH 9,0 durch Zugabe einer Base, und
iii. die Absenkung des pH-Wertes des in Schritt ii. erhaltenen Gemisches auf einen zweiten pH-Wert im Bereich von pH 6,5 bis pH 7,5 durch Zugabe einer Säure,
**dadurch gekennzeichnet, dass** der in Schritt i. verwendete Chelat-Komplexbildner mit der in Schritt iii. verwendeten Säure identisch ist.

2. Verfahren gemäß Anspruch 1, **dadurch gekennzeichnet, dass** das in Schritt i. erhaltene Gemisch im Schritt ii. auf einen ersten pH-Wert in einem Bereich von pH 7,9 bis pH 8,3 eingestellt wird und das in Schritt ii. erhaltene Gemisch im Schritt iii. auf einen pH-Wert in einem Bereich von pH 6,8 bis pH 7,4 eingestellt wird.

3. Verfahren gemäß Anspruch 1 oder Anspruch 2, **dadurch gekennzeichnet, dass** der in Schritt i. verwendete Chelat-Komplexbildner bzw. die in Schritt iii. verwendete Säure ausgewählt aus der Gruppe bestehend aus DOTA, DTPA und dessen Derivate, D03A und dessen Derivate oder BOPTA.

4. Verfahren gemäß Anspruch 3, **dadurch gekennzeichnet, dass** der Schritt i. verwendete Chelat-Komplexbildner bzw. die in Schritt iii. verwendete Säure DOTA ist.

5. Verfahren gemäß einem der vorherigen Ansprüche, **dadurch gekennzeichnet, dass** für die Durchführung von Schritt ii. die Base Meglumin verwendet wird.

6. Verfahren zur Herstellung von flüssigen zur parenteralen Verabreichung geeigneten pharmazeutischen Formulierungen enthaltend Gadolinium-DOTA mit reduziertem Gehalt an toxischen Verunreinigungen, umfassend die folgenden Schritte:
i. das Vermischen einer vorbestimmten Menge von Gadoliniumoxid mit einer vorbestimmten Menge DOTA in Wasser,
ii. das Einstellen des pH-Wertes des in Schritt i. erhaltenen Gemisches Gemisches auf einen ersten pH-Wert im Bereich von pH 7,9 bis pH 8,3 durch Zugabe von Meglumin, und
iii. Absenkung des pH-Wertes des in Schritt ii. erhaltenen Gemisches auf einen zweiten pH-Wert im Bereich von pH 6,8 bis pH 7,4 durch Zugabe von DOTA.

7. Flüssige zur parenteralen Verabreichung geeignete pharmazeutische Formulierung enthaltend einen Komplex eines Chelat-Komplexbildners mit Gadolinium, **gekennzeichnet dadurch, dass** der Gehalt an Zink kleiner als 50 ppm, der Gehalt an Arsen kleiner als 3 ppm, der Gehalt an Blei, Kobalt sowie Cadmium jeweils kleiner als 1,5 ppm und der Gehalt an Quecksilber kleiner als 1,0 ppm ist.

8. Flüssige zur parenteralen Verabreichung geeignete pharmazeutische Formulierung gemäß Anspruch 7, **gekennzeichnet dadurch, dass** der Gehalt an Zink kleiner als 10 ppm, bevorzugt kleiner als 5 ppm, ist.

9. Flüssige zur parenteralen Verabreichung geeignete pharmazeutische Formulierung gemäß Anspruch 7 oder 8, **gekennzeichnet dadurch, dass** der Gehalt an Arsen, Blei, Cadmium sowie Kobalt jeweils kleiner als 1,0 ppm, bevorzugt kleiner als 0,5 ppm, ist.

10. Flüssige zur parenteralen Verabreichung geeignete pharmazeutische Formulierung gemäß einem der Ansprüche 7 bis 9, **dadurch gekennzeichnet, dass** der pH-Wert der Formulierung in einem Bereich von pH 6,5 bis pH 7,5, bevorzugt in einem Bereich von pH 6,8 bis pH 7,4 liegt.

11. Flüssige zur parenteralen Verabreichung geeignete pharmazeutische Formulierung gemäß einem der Ansprüche 7 bis 10, **dadurch gekennzeichnet, dass** der Chelatausgewählt aus der Gruppe bestehend aus DOTA, DTPA und dessen Derivate, D03A und dessen Derivate oder BOPTA.

12. Flüssige zur parenteralen Verabreichung geeignete pharmazeutische Formulierung gemäß Anspruch 11, **dadurch gekennzeichnet, dass** die pharmazeutische Formulierung eine pharmazeutische Formulierung des Megluminsalzes des Gadolinium-DOTA-Komplexes ist.

13. Flüssige zur parenteralen Verabreichung geeignete pharmazeutische Formulierung enthaltend einen Komplex aus Gadolinium und DOTA sowie Meglumin, **dadurch gekennzeichnet, dass** der Gehalt an Zink kleiner als 50 ppm und der Gehalt an Arsen, Blei, Cadmium, Kobalt sowie Quecksilber jeweils kleiner als 1,0 ppm ist.

14. Verwendung einer flüssigen zur parenteralen Verabreichung geeigneten pharmazeutischen Formulierung gemäß einem der Ansprüche 7 bis 13 als Kontrastmittel für die Magnetresonanzbildgebung.
